# EUROPEAN PATENT APPLICATION

(11) **EP 3 382 583 A1**
(43) Date of publication of application: **03.10.2018**
(21) Application number: 17163524.6
(22) Date of filing: 29.03.2017
(51) Int. Cl.: G06F 19/00, A61B 6/00

(54) **HEMODYNAMIC SIMULATION OF MOVEMENT INDUCTED VASCULAR DEFORMATIONS**

(71) Applicant: Koninklijke Philips N.V., 5656 AE Eindhoven (NL)
(72) Inventor: HAASE, Christian, 5656 AE Eindhoven (NL); GRASS, Michael, 5656 AE Eindhoven (NL); SCHMITT, Holger, 5656 AE Eindhoven (NL); KAHLERT, Joachim, 5656 AE Eindhoven (NL)
(74) Representative: de Haan, Poul Erik

(57) **Abstract**

The present invention relates to an apparatus (1) and a method for determining one or more values of functional physiological parameters for a vessel structure around a joint structure of a living being in a non-invasive manner taking into account the impact of anatomical motion onto the vessel structure. A representation determination unit (11) is adapted to determine a deformation of a first representation of the vessel structure based on a deformation model and first and second postures of the joint structure, and to determine a second representation based on the first representation and the determined deformation. A parameter value determination unit (13) is adapted to determine a value of the functional physiological parameter based on a hemodynamical simulation model and the second representation. The apparatus thus provides for an improved severity classification of functional physiological parameter for a vessel structure.

## Description

### FIELD OF THE INVENTION

The invention relates to an apparatus, a method and a computer program for determining in a non-invasive manner a value of one or more functional physiological parameters for at least a portion of a vascular system of a living being. In particular, the invention relates to the determination of intravascular pressure gradients in peripheral arteries or veins.

### BACKGROUND OF THE INVENTION

The physiological significance of arterial disease can be classified using an intra-arterial pressure wire to measure established quantities like pressure gradients. Hemodynamic simulations and virtual intravascular pressure gradient calculation based on angiography data of diseased arteries provide a non-invasive method to derive important hemodynamic parameters. Even though hemodynamic simulations provide reliable disease classification in many cases, invasive measurements remain the gold standard for difficult and uncertain cases.

For arterial disease that is located at a specific location like e.g. in a vessel close to a joint, such as the knee, anatomical motion can be an important factor for the physiological evaluation since flexing the knee may cause shortening and strain of a vessel in that region and change the physiological impact and thus the measured hemodynamic parameters of the disease.

In particular two cases are of clinical importance, the coiling of the carotids when the head is flexed or bent and the kinking of the iliac arteries. Cyclists may have an insufficient blood flow under vigorous cycling due to the body posture. Angiographic data may show the vessel structure and the kinking probabilities, but it is not possible to estimate if the blood flow will be sufficient when the blood flow has to be increased by a factor of ten during vigorous exercising.

### SUMMARY OF THE INVENTION

It is an object of the present invention to provide an apparatus, a method and a computer program for determining one or more values of functional physiological parameters of a living being, which allows for a non-invasive determination of these one or more values with improved severity detection capability.

In a first aspect of the present invention an apparatus for determining a value of a functional physiological parameter of a living being is presented. The apparatus comprises:
an image data providing unit for providing image data showing a vessel structure around a joint structure in a first posture;
a segmentation unit for segmenting the vessel structure in the image data to generate a first representation of the vessel structure;
a posture providing unit for providing the first posture of the joint structure and a second posture of the joint structure;
a representation determination unit for determining a second representation of the vessel structure, which is present when the joint structure is in the second posture, wherein the representation determination unit is adapted to provide a deformation model defining a deformation of a representation of a vessel structure in dependence of a posture change of a joint structure, to determine a deformation of the first representation based on the deformation model and the first and second postures of the joint structure, and to determine the second representation based on the first representation and the determined deformation; and
a parameter value determination unit for determining the value of the functional physiological parameter, wherein the parameter value determination unit is adapted to provide a hemodynamical simulation model defining a value of a functional physiological parameter in dependence of a representation of a vessel structure and to determine the value of the functional physiological parameter based on the hemodynamical simulation model and the second representation.

Anatomical motion can be an important factor for the physiological evaluation of a vascular disease that is located close to a highly flexible region, such as a joint. For instance, flexing the knee may cause shortening and strain of a vessel of the vascular system and may change the physiological impact and thus the measured hemodynamic parameters of a disease in the area of the knee joint. Since the representation determination unit can determine any desired deformed representation of the vessel structure around the joint structure, the parameter value determination unit can determine the value of the functional physiological parameter for a desired arbitrary posture of the joint structure in a non-invasive manner and thus improve the severity detection capability. By incorporating anatomical motion in hemodynamic simulations, the claimed apparatus can assist in a virtual physiological disease classification. Additional to the advanced virtual disease classification, the information obtained by these hemodynamic simulations can be used to provide advice for an optimal and consistent invasive measurement of hemodynamic parameters.

The image data providing unit can comprise a storing unit for storing already captured image data of the vessel structure and for providing the stored image data. The image data providing unit can also comprise a receiving unit for receiving already generated image data of the vessel structure and for providing the received image data. Moreover, the image data providing unit can be adapted to generate the image data of the vessel structure and to provide the generated image data. The image data may be obtained from any imaging modality capable of providing information about the vessel structure and preferentially about the bone structure, e.g. a CT data set, an X-ray data set, an ultrasound data set or a combination thereof.

The segmentation unit segments the provided image data to extract the vessel structure using known segmentation techniques, such as but not limited to edge detection, clustering methods, region growing methods, etc. The most appropriate segmentation technique may depend on the type of image data provided by the image data providing unit. The segmentation unit generates a first representation of the vessel structure. Preferentially the first representation is a 3D-model of the vessel structure.

The first and second joint posture providing units provide information regarding the first and second postures of the joint structure, respectively. The first joint posture corresponds to the posture of the joint structure in the image data. It may be provided externally, e. g.by manual input, or it may be derived from technical parameters received from a device that is used to take the image data. The second joint posture may be any posture of the joint structure that is of interest to a user. It may be entered by a user, e.g. selected from a predefined drop down list or entered via a keyboard or any other input means.

The representation determination unit can generate a representation of the vessel structure corresponding to the second posture of the joint structure using a deformation model that describes deformations of a vessel structure in dependence of a posture change of a joint structure. The deformations, in particular average changes to vessel length and curvature that typically occur during bending and/or twisting of a joint structure can be derived from biomechanical joint models or from MR based studies as disclosed in the article "Quantification of popliteal artery deformation during leg flexion in subjects with peripheral artery disease: a pilot study" by Gökgöl, Can, et al., Journal of Endovascular Therapy, volume 20, issue 6, pages 828-835 (2013) which is herewith incorporated by reference.

Alternatively, a statistical model or a biomechanical finite element model may be used as deformation model to determine deformations like radial compression, bending, torsion, axial extension and axial compression of the vascular system. Such models are inter alia described in "Effects of knee flexion on the femoropopliteal artery: A computational study" by Ghriallais, Riona Ni, and Mark Bruzzi, Medical engineering & physics, volume 35, number 11 , pages 1620-1628 (2013) which is herewith incorporated by reference.

The parameter value determination unit can determine the value of the functional physiological parameter by using a hemodynamical simulation model that determines the value of the functional physiological parameter based on the provided deformed second representation of the vessel structure. Hemodynamical simulation models can be used to determine a variety of functional physiological parameters. Simulation techniques are disclosed, inter alia, in the articles "Diagnosis of ischemia-causing coronary stenoses by noninvasive fractional flow reserve computed from coronary computed tomographic angiograms. Results from the prospective multicenter DISCOVER-FLOW (Diagnosis of Ischemia-Causing Stenoses Obtained Via Noninvasive Fractional Flow Reserve) study" by B.K. Koo et al., Journal of the American College of Cardiology, 58 (19), pages 1989 to 1997 (2011), "Patient-specific modeling of blood flow and pressure in human coronary arteries" by H. J. Kim et al., Annals of Biomedical Engineering 38(10), pages 3195 to 3209 (2010), and "Outflow boundary conditions for three-dimensional simulations of non-periodic blood flow and pressure fields in deformable arteries" by I. E. Vignon-Clementel et al., 13(5), pages 625 to 640 (2010), which are herewith incorporated by reference.

A functional physiological parameter may be any parameter describing the vascular system, such as blood flow velocity, blood pressure, and/or blood temperature, but also more complex parameters derived from one or more basic parameters, such as intravascular pressure gradients. The intravascular pressure gradient is one of the most relevant and well established indicators of vascular health. The intravascular pressure gradient provides an indication of the pressure drop between two positions. The pressure after (distal to) a stenosis usually differs from the pressure before the stenosis. The pressure gradient, that is the change in pressure, provides estimation for the severity of the stenosis. The pressure gradient may be compared to the pressure before a stenosis. If the pressure does not change, the pressure gradient would be 0. The larger the pressure drop, the closer the value of the pressure gradient normalized by the pressure before the stenosis is to 1. Using both the first and second representation of the vessel structure, two separate fluid dynamics evaluations can be performed and the two different outcomes of e.g. the pressure drop along the vessel of interest can be presented to the user. This allows a better classification of disease severity since e.g. a lesion that may not have an impact for a straight leg, may have significant functional impact in the flexed state.

In an embodiment the segmentation unit is further configured to segment the joint structure from the image data and the first posture providing unit is configured to determine the first posture based on the segmented joint structure. This embodiment provides the further advantage that the first posture of the joint structure can be deduced automatically which provides a higher precision and requires no manual input. The image data may comprise both, information regarding the vessel structure as well as information regarding the bone structure (CT data). Furthermore, the image data may also comprise data from more than one image source to provide both information on the vessel as well as the joint structure. The joint structure may be segmented from the image data using known segmentation techniques, such as but not limited to edge detection, clustering methods, region growing methods, etc. The most appropriate segmentation technique may depend on the type of image data provided by the image data providing unit. In the visual presentation of the 3D-model of the vessel structure the joint structure may be controllably displayed in addition. From the segmented joint structure, the first posture providing unit may determine the anatomical posture of the joint, e.g. but not limited to - by identifying characteristic points and/or by comparison with reference joint structures. Again, the first posture providing unit may alternatively or in addition use further information provided externally either manually by a user or from other data associated with the image data, e.g. technical parameters from an imaging device providing the image data, wherein the technical parameters may indicate whether the living being was lying straight, sitting, having arms or legs in a particular position, etc. while the image data had been generated.

In an embodiment of the present invention, the second posture providing unit is adapted to provide several second postures of the joint structure defining a full motion cycle of the joint structure, the representation determination unit is configured to determine a set of second representations of the vessel structure for the several second postures, and the parameter value determination unit is configured to determine a set of respective values of the functional physiological parameter based on the provided set of second representations of the vessel structure.

A full motion cycle of the joint structure may be any movement of the joint from a particular starting position to a particular end position. For example, if the region of interest is the knee joint or the ankle, then a full motion cycle could be the motion of the knee during walking, cycling or sitting down. If the region of interest is the anatomical region around the shoulder or neck, a full motion cycle may be lifting an arm, turning the head left/right or up/down.

Preferentially, the parameter value determination unit may further be configured to select the value of the functional physiological parameter out of the set of values of the functional physiological parameter that differs maximally from a preset value. In order to ease the assessment of the set of values determined for the set of second representations, a reference value may be defined, for instance, a normalized intravascular pressure gradient of 0, which indicates no pressure loss. The parameter value determination unit may then automatically select the largest normalized intravascular pressure gradient value furthest away from the reference value or all values deviating by a predetermined amount determined within the set of values corresponding to the set of deformed representations. This automatic preselecting might ease the assessment of the determined values and thus can help to provide a diagnosis.

The apparatus may further comprise an output unit for outputting the second posture of the joint structure corresponding to the selected value of the functional physiological parameter that differs maximally from the present value. That way, an advice can be given to a physician in which position the most significant functional effect of a potential disease can be expected. The physician may then subsequently perform an invasive physiological measurement directly in the most relevant state, e.g. having the joint in the position corresponding to the detected value.

The output unit may further output a respective value of the set of values of the functional physiological parameter together with a representation of the corresponding vessel structure preferably including the joint structure in the corresponding position. Preferentially, the output unit may also display a corresponding indication of the posture of the human being in which the determined joint posture usually occurs. By outputting a paired display of the value of the functional physiological parameter and the corresponding joint posture or posture of the human being, a user of the apparatus may scroll through the paired displays of a set of values which eases the assessment of the severity classification and helps to identify outlier. If, for instance, an intravascular pressure gradient value in a row of representations has a similar value indicating a stenosis, the reliability of that value is high and the disease is relevant in a variety of joint positions. If, on the other hand, an intravascular pressure gradient value differs significantly from the previous and next intravascular pressure gradient in a row of representations corresponding to a full motion cycle, the intravascular pressure gradient value may be an outlier. Further investigations should be initiated. If the intravascular pressure gradient value is confirmed, the joint posture corresponding to the identified intravascular pressure gradient value might be identified and avoided as much as possible.

In a further embodiment the hemodynamical simulation model is adapted to define a value of a functional physiological parameter in dependence of a representation of a vessel structure and also in dependence of an activity level parameter being indicative of an activity level of a living being, wherein the apparatus further comprises an activity parameter providing unit for providing an activity parameter being indicative of the activity level of the living being, wherein the parameter value determination unit is configured to determine the value of the functional physiological parameter based on the hemodynamical simulation model, the second representation and the provided activity parameter. The activity parameter may be one or more vital parameters like for instance - but not limited to - blood pressure (preferably non-invasively measured with an inflatable blood pressure cuff), heartbeat frequency, and temperature. The activity parameter providing unit may receive an activity parameter from storage or from an input unit, wherein the input unit may either receive a manual input or measurement data from an external device to measure an activity parameter while the living being is at rest and/or while vigorously exercising.

Hemodynamical simulation models may use on or more activity parameters to determine a value of the functional physiological parameter for different stress scenarios. For example, the blood flow through the vessel structure may differ significantly for a living being at rest and a living being under vigorous exercising. While it is difficult to perform invasive measurements under vigorous exercising conditions, a simulation may provide a quick estimate of the hemodynamics and the functional physiological parameters derivable thereof with slightly changed boundary conditions regarding the activity parameters provided to the simulation model.

In a further embodiment the posture providing unit is further adapted to provide a third posture of the joint structure, the image data providing unit is adapted to provide second image data showing the vessel structure around the joint structure in the third posture, the segmentation unit is adapted to segment the vessel structure in the second image data to generate a third representation of the vessel structure, and the deformation determination unit is adapted to provide the deformation model by interpolating the vessel structure between the first and third representations.

The interpolation between two known representations of the vessel structure corresponding to two different postures of the joint structure might present a simple approach to provide a deformation model. Corresponding feature points in both representations may be identified, e.g. end positions of bones or vessels, branching connections, etc., and a linear interpolation might be performed between the two positions of the feature points to define a motion vector field that describes a continuous transition from the first to the third representation.

In a further aspect of the present invention a method for determining a value of a functional physiological parameter of a living being is presented, the method comprising:
providing image data showing a vessel structure around a joint structure in a first posture;
segmenting the vessel structure in the image data to generate a first representation of the vessel structure;
providing the first posture of the joint structure;
providing a second posture of the joint structure;
determining a second representation of the vessel structure, which is present when the joint structure is in the second posture, by providing a deformation model defining a deformation of a representation of a vessel structure in dependence of a posture change of a joint structure, and determining a deformation of the first representation based on the deformation model and the first and second postures of the joint structure, and to determine the second representation based on the first representation and the determined deformation;
determining the value of the functional physiological parameter by providing a hemodynamical simulation model defining a value of a functional physiological parameter in dependence of a representation of a vessel structure and by determining the value of the functional physiological parameter based on the hemodynamical simulation model and the second representation.

In a further aspect of the present invention a computer program for determining a value of a functional physiological parameter of a living being is presented, the computer program comprising program code means for causing an apparatus as defined in claim 1 to carry out the steps of the method as defined in claim 10, when the computer program is run on a computer controlling the apparatus.

It shall be understood that the apparatus for determining a value of a functional physiological parameter of a living being of claim 1, the method for determining a value of a functional physiological parameter of a living being of claim 10, and the computer program for determining a value of a functional physiological parameter of a living being of claim 11 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 schematically and exemplarily shows an embodiment of an apparatus for determining a value of a functional physiological parameter in at least a part of the vascular system of a living being,
Fig. 2 schematically and exemplarily shows a diseased vessel structure in a region of a knee joint at 0°, 45° and 90° bending of the knee.
Fig. 3 schematically and exemplarily illustrates a flowchart of an embodiment of a determination method for determining a value of a functional physiological parameter in at least a part of a vascular system of a living being,

### DETAILED DESCRIPTION OF EMBODIMENTS

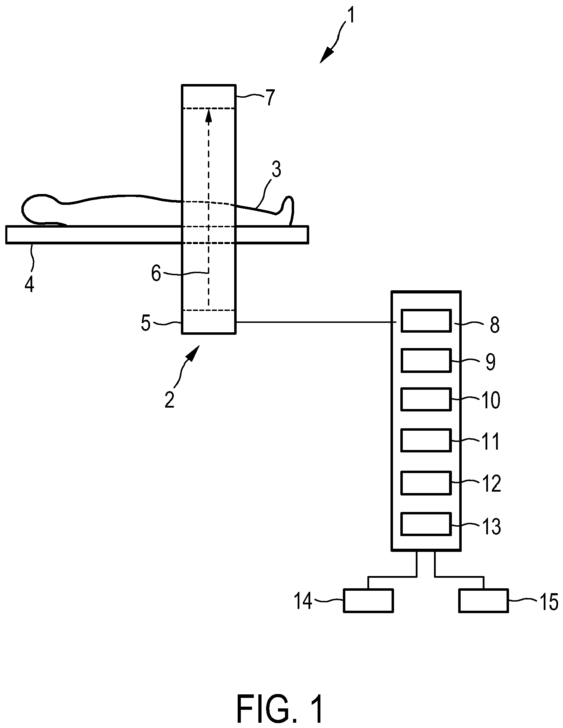

Fig. 1 shows schematically and exemplarily an embodiment of an apparatus 1 for determining a value of a functional physiological parameter in a vessel structure of a living being 3. In this embodiment the apparatus 1 comprises a projection data generating unit 2 for providing projection data having been generated by a detector 7 over time based on radiation 6, which is generated by a radiation source 5 and which has traversed a vessel of the vascular system or a region of vessels of the vascular system of the person 3 lying on a support means 4 like a table. The projection data generating unit 2 is preferentially an acquisition unit of a CT system. The apparatus 1 can therefore also be regarded as being a CT system which is adapted to determine a value of a functional physiological parameter in a vessel structure of the living being 3.

The apparatus 1 further comprises an image data providing unit 8 for providing image data of the vessel structure around a joint structure in a first posture. In this embodiment the image data providing unit 8 is adapted to reconstruct a set of CT images showing a vessel structure based on the acquired projection data. The image data providing unit 8 may alternatively or in addition receive image data from storage of either a remote server or a local memory.

The apparatus 1 further comprises a segmentation unit 9 to segment the vessel structure in the reconstructed set of CT images, in order to provide a representation of the vessel structure. The segmentation unit 9 maybe adapted to use, for instance, a model-based segmentation approach, a region growing or wave propagation method or an interactive vascular segmentation technique for segmenting the region of the vascular system. The resulting representation is preferentially a three-dimensional representation of the region of the vessel structure.

The segmentation unit 9 in this embodiment further segments the joint structure in the image data provided by the image data providing unit 9 and identifies a corresponding anatomical posture of the joint structure in which the living being 3 remained while the image data was recorded. In this embodiment, the segmentation unit 9 identifies the bone and in particular the joint structure from the reconstructed CT images. Since the person 3 is lying straight on the support means 4, the first posture will correspond to a straight or at least almost straight knee joint.

A posture providing unit 10 determines a first posture of the knee joint by identifying characteristic feature points in the segmented joint structure. Alternatively or in addition, the posture providing unit 10 may receive manual input via input unit 14 which may be provided by the clinician or which may be derived from technical parameters recorded together with the image data indicative of the position in which the joint structure was recorded.

Via the input means 14 an arbitrary second posture may be selected which is then provided by as second posture by the posture providing unit 10.

The apparatus 1 further comprises a representation determination unit 11 for providing a second representation of the vessel structure around the joint structure corresponding to the selected second posture provided by the posture providing unit 10. A knee joint will most often be imaged in a straight leg position as indicated in Fig. 1. However, it might be interesting to determine the impact of a stenosis observed in the region of the knee joint in a bent state. The representation determination unit 11 in this embodiment uses a deformation model being a statistical motion model that describes expected deformations of the vessel structure around a joint region. Alternatively, average changes to vessel length and curvature in the vascular system may be derived from a biomechanical joint model, magnetic-resonance based studies or a biomechanical finite element model. The representation determination unit 11 determines a deformation of the vessel structure in the first representation expected for a change from the first posture to the second posture of the joint structure. For instance, taking the statistical model average changes to the vessel structure and vessel curvature corresponding to the change between first and second posture would be added to the first representation to obtain the second representation.

The apparatus 1 further comprises a parameter value determination unit 13 for determining a value of the functional physiological parameter based on the provided second representation of the vessel structure. In particular, the parameter value determination unit 13 may be adapted to simulate the flow of blood within the vessel structure based on the second representation of the vessel structure to determine for instance the intravascular pressure gradient value based on the simulated flow of the blood, wherein the intravascular pressure gradient value can be determined such that it is indicative of a blood pressure drop across a vessel-narrowing stenosis. The intravascular pressure gradient value of the vessel structure may be defined as the blood pressure drop across a vessel-narrowing stenosis. The parameter value determination unit 13 is preferentially adapted to simulate vascular fluid dynamics and to determine functional physiological parameters such as the intravascular pressure gradient value based on the provided representation of the vessel structure, in order to determine the intravascular pressure gradient value non-invasively. Optionally, the apparatus may comprise an activity parameter providing unit 12 to provide one or more activity parameters corresponding to one or more stress levels of the living being 3. The activity parameter may be one or more vital parameters like for instance - but not limited to - blood pressure (preferably non-invasively measured with an inflatable blood pressure cuff), heartbeat frequency, and temperature. The activity parameter providing unit 12 may receive an activity parameter from storage or from input means 14, wherein the input means 14 may either receive a manual input or measurement data from an external device to measure an activity parameter while the living being 3 is at rest and/or while vigorously exercising.

The input unit 14 can be a keyboard, speech recognition, a computer mouse, a touch pad, et cetera for allowing a user to input commands like a start command or a stop command into the apparatus 1, or to set parameters defining, for instance, the acquisition of image data, the segmentation of the vascular system, the anatomical position of the joint, the determination area of the intravascular pressure gradient values, the vital parameters of the living being 3 for different stress scenarios, e.g. rest or vigorous exercising, et cetera. The vital parameters may also be provided automatically by connected measurement devices. The apparatus 1 also comprises an output unit 15 providing visual and/or acoustic output for showing, for instance, a generated CT image of vessel structure of interest, the provided representation of the vessel structure, a determined parameter value or set of values, e.g. intravascular pressure gradient, a joint posture corresponding to the one or more parameter values et cetera. The output unit 15 may provide a display unit wherein the display unit may either be part of the output unit 15 as a fixed part of the apparatus or the display may be a handheld device such as a portable tablet which receives its data wirelessly, e.g. Bluetooth or RF signaling.

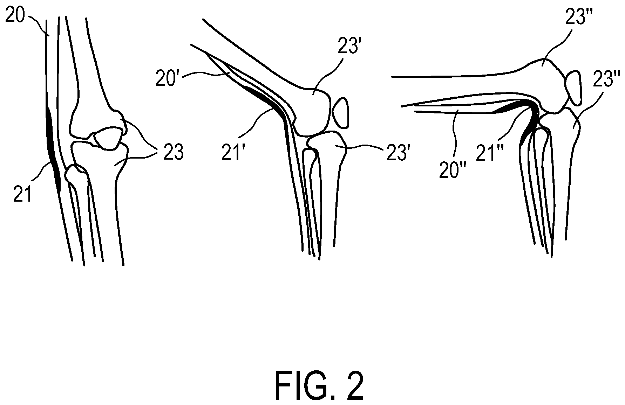

The apparatus 1 may thus determine intravascular pressure gradient values for a vessel 20 positioned for instance close to the knee joint 23 as illustrated schematically and exemplarily in Fig. 2. Wherein a stenosis 21 may not impact the blood flow significantly when the knee joint 23 is in a straight posture, the vessel 20' might change its geometry if the knee joint 23' is bent by 45° and even further if the knee joint 23" is bent by 90°. The stenosis 21' in the vessel 20' at 45° and the stenosis 21" in the vessel 20" at 90° bending of the knee joint might have a significant larger functional impact than the same stenosis 21 in the straight leg.

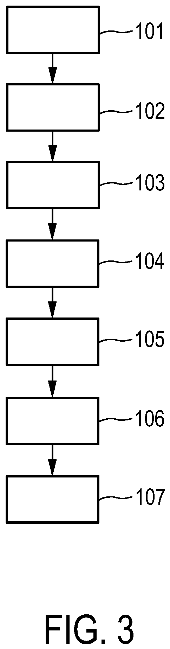

Fig. 3 shows schematically and exemplarily an embodiment of a method for determining a value of a functional physiological parameter of a living being. Upon initiation of the method first image data of the vessel structure around a joint structure in a first posture is provided 101, either from memory or from real time imaging by any imaging modality suitable for imaging the vascular system and/or the bone structure, e.g. - but not limited to - X-ray, CT, ultrasound, etc.. In a next step 102, the vessel structure is segmented from the provided image data to generate a first representation of the vessel structure, preferentially as a 3D-model. Optionally, if the image data also comprises data regarding the bone structure, a joint structure is segmented in step 102 as well. In step 103, the first posture of the joint structure is provided. If the joint structure has been segmented as well in step 102, the identification may be performed automatically by analyzing characteristic feature points of the joint structure indicative of the actual posture of the joint in which the image data was recorded. This identification may for instance be based on imaging techniques capable of learning from previously recorded image data and identifying a bone/joint structure using characteristics typical for respective anatomical areas. If the joint structure is not segmented or no automatic detection is possible, the first posture may be input externally, e.g. by a clinician via input means 14 or derived from technical specifications of the projection data generating unit 2 which specify the joint position. In a further step 104, a second posture of the joint structure is provided. A user may select the second posture from a list or enter a bending angle via the input means 14. In step 105, a second representation of the vessel structure corresponding to the second posture of the joint structure is determined. A deformation model is provided which describes a deformation of a representation of a vessel structure in dependence of a posture change of a joint structure. Average changes to vessel length and curvature that typically occur during knee flexion, hip flexing or neck bending can for instance be deduced from biomechanical joint models or from MR based studies and applied to the first representation. Using a large number of data sets with segmented arteries and/or veins for bent and straight knee positions, a statistical procedure like principle component analysis can be used to identify the major deformation modes. Alternatively, a biomechanical finite element model maybe used to determine deformations like radial compression, bending, torsion, axial extension and axial compression. The determined deformations are then applied to the first representation to determine the second representation.

In a further step 106, a value of the a functional physiological parameter, in particular the intravascular pressure gradient, is determined using a hemodynamical simulation model defining a value of a functional physiological parameter in dependence of a representation of a vessel structure based on the provided second representation of the vessel structure. The same value may be determined for the first representation to provide a comparison. Using both the first and second representation two separate fluid dynamics evaluations are performed and the two different outcomes of e.g. the pressure drop along the vessel of interest may be presented to the user. This allows better classification of disease severity since e.g. a lesion that may not have an impact for a straight joint, may have significant functional impact in the flexed state. An advice may be given to a physician in which leg position the more significant functional effect of the disease can be expected. The physician can then perform an invasive physiological measurement directly in the more relevant state. The steps 105 and 106 may be performed several times, either parallel or iteratively, to generate a set of second representations and determine respective values of the functional physiological parameter. The set of second representations may correspond to a full motion cycle of a respective movement. If the knee joint is the anatomical region of interest, a full motion cycle may refer to the movement of the knee during walking, running or cycling. The hemodynamical simulation model may then be used to determine functional physiological parameter, in particular the intravascular pressure gradient, for each representation of the motion cycle. If a simple steady state flow condition in a first vessel geometry is simulated, the result will be a well-defined functional parameter independent of a point in time which only depends on the present geometry. If however, the geometry is constantly changing, the functional parameter for the first geometry may also be expressed taking into account the preceding vessel geometry (which was present in the time interval before). For instance, if geometry 0 is a total occlusion and geometry 1 is a 60% diameter narrowing and if geometry 1 exists only for a short time, the expected blood flow would be particularly small since some initial acceleration effects would need to be taken into account. A time-dependent dynamic fluid model (and time dependent geometry) may be used to take these effects into account. The resulting parameter may then be expressed as time dependent value or as an average functional parameter (e.g. total blood flow volume) over the full periodic cycle. In order to simulate the impact of for instance vigorous exercising, the hemodynamical simulation model may allow adapting flow parameters, e.g. accelerated heartbeat, increased body temperature, et cetera which in addition or in combination with certain body postures might impact the severity of arterial diseases. The adaptation of the flow parameters might be achieved by an extrapolation of measured flow parameters at rest. Alternatively, the flow parameters may be obtained in several measurements in which the vital parameters of a living being are monitored and recorded during different levels of stress, e.g. rest and vigorous exercising. The obtained parameters may then be provided to the hemodynamical simulation model as additional boundary conditions where they can be combined with a representation of the vessel structure of interest. This is in particular advantageous since invasive pressure measurements cannot be performed during vigorous exercising. Sometimes drug induced stress testing may be performed wherein invasive pressure measurements are performed at a patient who previously received a drug like adenosine in order to simulate stress. However, it is not possible to take into account motion induced effects in drug induced stress testing. The ability to determine a dynamic flow restriction during a periodic exercise like walking can only be obtained by a simulation since it can combine the different levels of restriction that occur during a cycle into an overall result. A single measurement may not be able to provide the same level of detail for a subsequent diagnosis.

Procedures like providing image data, segmenting bone and vessel structures, identifying joint structures, providing representations, et cetera performed by one or several units or devices can be performed by any other number of units or devices. These procedures and/or the control of the method for determining a value of a functional physiological parameter of a living being can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. An apparatus (1) for determining a value of a functional physiological parameter of a living being (3), wherein the apparatus (1) comprises:
an image data providing unit (8) for providing image data showing a vessel structure around a joint structure in a first posture;
a segmentation unit (9) for segmenting the vessel structure in the image data to generate a first representation of the vessel structure;
a posture providing unit (10) for providing the first posture of the joint structure and for providing a second posture of the joint structure;
a representation determination unit (11) for determining a second representation of the vessel structure, which is present when the joint structure is in the second posture, wherein the representation determination unit (11) is adapted to provide a deformation model defining a deformation of a representation of a vessel structure in dependence of a posture change of a joint structure, to determine a deformation of the first representation based on the deformation model and the first and second postures of the joint structure, and to determine the second representation based on the first representation and the determined deformation;
a parameter value determination unit (13) for determining the value of the functional physiological parameter, wherein the parameter value determination unit (13) is adapted to provide a hemodynamical simulation model defining a value of a functional physiological parameter in dependence of a representation of a vessel structure and to determine the value of the functional physiological parameter based on the hemodynamical simulation model and the second representation.

2. The apparatus (1) according to claim 1, wherein the segmentation unit (9) is further configured to segment the joint structure from the image data and the posture providing unit (10) is configured to determine the first posture based on the segmented joint structure.

3. The apparatus (1) according to claim 1, wherein
the posture providing unit (10) is adapted to provide several second postures of the joint structure defining a full motion cycle of the joint structure;
the representation determination unit (11) is configured to determine a set of second representations of the vessel structure for the several second postures; and
the parameter value determination unit (13) is configured to determine a set of respective values of the functional physiological parameter based on the hemodynamical simulation model and the provided set of second representations of the vessel structure.

4. The apparatus (1) according to claim 3, wherein the parameter value determination unit (13) is further configured to select the value of the functional physiological parameter out of the set of values of the functional physiological parameter that differs maximally from a preset value.

5. The apparatus (1) according to claim 4, further comprising an output unit (15) for outputting the second posture of the joint structure corresponding to the selected value of the functional physiological parameter that differs maximally from the present value.

6. The apparatus (1) according to claim 1, wherein the hemodynamical simulation model is adapted to define a value of a functional physiological parameter in dependence of a representation of a vessel structure and also in dependence of an activity level parameter being indicative of an activity level of a living being, wherein the apparatus (1) further comprises an activity parameter providing unit (12) for providing an activity parameter being indicative of the activity level of the living being (3), wherein the parameter value determination unit (13) is configured to determine the value of the functional physiological parameter based on the hemodynamical simulation model, the second representation and the provided activity parameter.

7. The apparatus (1) according to claim 1, wherein the functional physiological parameter is an intravascular pressure gradient.

8. The apparatus (1) according to claim 1, wherein the deformation model is at least one of a biomechanical joint model, a statistical model, a biomechanical finite element model, or a model derived from magnetic-resonance based studies.

9. The apparatus (1) according to claim 1, wherein
the posture providing unit (10) is adapted to provide a third posture of the joint structure;
the image data providing unit (8) is adapted to provide second image data showing the vessel structure around the joint structure in the third posture;
the segmentation unit (9) is adapted to segment the vessel structure in the second image data to generate a third representation of the vessel structure; and
the representation determination unit (11) is adapted to provide the deformation model by interpolating between the first and third representations.

10. A method for determining a value of a functional physiological parameter of a living being (3), the method comprising:
providing (101) image data showing a vessel structure around a joint structure in a first posture;
segmenting (102) the vessel structure in the image data to generate a first representation of the vessel structure;
providing (103) the first posture of the joint structure;
providing (104) a second posture of the joint structure;
determining (105) a second representation of the vessel structure, which is present when the joint structure is in the second posture, by providing a deformation model defining a deformation of a representation of a vessel structure in dependence of a posture change of a joint structure, and determining a deformation of the first representation based on the deformation model and the first and second postures of the joint structure, and to determine the second representation based on the first representation and the determined deformation;
determining (106) the value of the functional physiological parameter by providing a hemodynamical simulation model defining a value of a functional physiological parameter in dependence of a representation of a vessel structure and by determining the value of the functional physiological parameter based on the hemodynamical simulation model and the second representation.

11. A computer program for determining a value of a functional physiological parameter of a living being (3), the computer program comprising program code means for causing an apparatus (1) as defined in claim 1 to carry out the steps of the method as defined in claim 10, when the computer program is run on a computer controlling the apparatus (1).
